# EUROPÄISCHE PATENTSCHRIFT

(11) **EP 1 582 594 B1**
(45) Veröffentlichungstag und Bekanntmachung des Hinweises auf die Patenterteilung: **11.03.2009**
(21) Anmeldenummer: 05006205.8
(22) Anmeldetag: 22.03.2005
(51) Int. Cl.: C12P 7/64

(54) **Verfahren zur beschleunigten enzymatischen Synthese von Triglyzeriden ungesättigter Fettsäuren**
Enzymatic process for the accelerated synthesis of triglycerides containing polyunsaturated fatty acid
Procédé enzymatique accéléré de synthèse de triglycérides comprenant des acides gras polyinsaturés

(30) Priorität: 31.03.2004 DE 102004015781
(43) Veröffentlichungstag der Anmeldung: 05.10.2005
(73) Patentinhaber: Cognis IP Management GmbH, 40589 Düsseldorf (DE)
(72) Erfinder: Schörken, Ulrich, 40591 Düsseldorf (DE); Meyer, Carolin, 40589 Düsseldorf (DE); Both, Sabine, 41470 Neuss (DE); Horlacher, Peter, 89287 Bellenberg (DE)

(56) Entgegenhaltungen:
- EP-A- 1 174 416
- EP-A- 1 582 595
- WO-A-00/49117
- WO-A-02/24935
- WO-A-91/16443
- WO-A-96/37587
- WO-A-99/47135
- DE-A- 4 222 374
- US-A- 5 288 619
- BORG, P. ET AL.: "Comparison between two processes for the enzymatic synthesis of tri-docosahexaenoylglycerol in a solvent-free medium" BIOTECHNOLOGY LETTERS, Bd. 22, 2000, Seiten 777-781, XP002340871
- CASTILLO E. ET AL: 'The Role of Silica Gel in Lipase-Catalyzed Esterification Reactions of High-Polar Substrates' JOURNAL OF THE AMERICAN OIL CHEMISTS' SOCIETY Bd. 74, Nr. 2, 1997, Seiten 77 - 85, XP008081166

## Beschreibung

Die Erfindung befindet sich auf dem Gebiet der Fettsäureester und betrifft ein neues Verfahren zur enzymatischen Synthese von Triglyceriden, die mehrfach ungesättigte Fettsäuren enthalten, das sich durch eine beschleunigte Umsetzung auszeichnet.

### Stand der Technik

Die Herstellung von Estern mehrfach ungesättigter Fettsäuren kann auf chemischem Wege wie auch auf enzymatischem Wege erfolgen. Chemische Synthesen haben den Nachteil, dass in der Regel sehr hohe Temperaturen eingesetzt werden müssen und große Mengen an basischen Katalysatoren benötigt werden, wodurch in höherem Maße Nebenprodukte und unerwünschte Isomerisierungen auftreten. Ein möglicher Weg zur Reduktion der Reaktionstemperatur bei der chemischen Synthese von Glycerolestern mehrfach ungesättigter Fettsäuren wurde in der Europäischen Patentanmeldung EP 1354 934 A1 offenbart. Durch den Einsatz eines Misch-Katalysators aus einem Salz einer schwachen Säure und einer starken Base zusammen mit der Seife einer organischen Säure mit 2 bis 26 Kohlenstoffatomen konnte die Reaktionstemperatur auf unter 175 °C, vorzugsweise 100 bis 140°C gesenkt werden.
Enzymatisch katalysierte Umsetzungen mit Lipasen finden jedoch dennoch in der Regel unter milderen Bedingungen statt und ergeben Endprodukte hoher Reinheit.

So werden in der Europäischen Patentschrift EP 0 950 410 A1 und auch der Internationalen Patentschrift WO 0 178 531 Synthesen beschrieben, in denen Glycerol und freie konjugierte Linolsäure (CLA) mit immobilisierter Lipase bei 65 °C und einem Vakuum von 0,01 bis 0,5 Torr zu CLA-triglyceriden umgesetzt werden. Ebenso wird gemäß der Europäischen Patentanmeldung EP 1174416 A1 Glycerol und freie CLA mit Lipase bei 70°C unter vermindertem Druck umgesetzt.
In der Europäischen Patentanmeldung EP 1 322 776 A1 wird eine lipase katalysierte Methode zur Herstellung von Triglyceriden mehrfach ungesättigter konjugierter Fettsäuren ausgehend vom Alkylester der ungesättigten Fettsäuren und Glycerin beschrieben, die den entstehenden Alkohol unter vermindertem Druck aus der Reaktion entfernt. Auch aus der Internationalen Patentanmeldung WO 9116443 A1 ist die Veresterung von Glycerol und freien mehrfach ungesättigten Fettsäuren oder deren Alkylestern zu den entsprechenden Triglyceriden durch Entfernung des Reaktionswassers bzw. des entstehenden Alkohols unter vermindertem Druck beschrieben.

US-A 5,288,619 offenbart ein Verfahren zur Lipase-katalysierten Synthese von Triglyceriden mit mehrfach ungesättigten Fettsäuren, bei der die Umsetzung der mehrfach ungesättigten Fettsäuren und/oder deren C₁-C₄₋Alkylestern mit Glycerin unter Vakuum in die entsprechenden Triglyceride durch Zugabe des Schleppmittels Stickstoffgas beschleunigt wird.

Castillo, E. et al.: The role of silica gel in lipase-catalyzed esterification reactions of high-polar substrates, Journal of the American oil chemists' society, Bd. 74, Nr. 2, 1997, Seiten 77-85, offenbart ein Verfahren zur Lipase-katalysierten Synthese von Triglyceriden mit mehrfach ungesättigten Fettsäuren, bei der die Umsetzung von Oleinsäure mit Glycerin in das entsprechende Triglycerid durch Zugabe des Glycerin bindenden Adsorbers Silicagel bzw. durch Zugabe des schwach basischen Ionenaustauschers Duolite^{™} A568 beschleunigt wird.

Enzymatische Synthesen haben jedoch häufig den Nachteil, dass die Umsetzungen relativ langsam ablaufen.

Die Aufgabe der vorliegenden Erfindung hat daher darin bestanden, enzymatische Verfahren zur Herstellung von Triglyceriden mit mehrfach ungesättigten Fettsäuren in ihrer Rentabilität zu verbessern.

Gegenstand der Erfindung ist ein Verfahren zur enzymkatalysierten Herstellung von Triglyceriden, die mehrfach ungesättigte Fettsäuren enthalten, bei dem man
(a) die Umsetzung von mehrfach ungesättigten Fettsäuren und/oder deren C₁ bis C₄-Alkylestern mit Glycerin unter Vakuum in ihre Triglyceride in Gegenwart eines Enzyms durch
   (i) Zugabe eines Additivs aus der Gruppe der schwach basischen Salze, Komplexierungsmittel und Salzen von sauren Ionenaustauschern und/oder
   (ii) Zugabe eines Schleppmittels in Form eines Lösungsmittels, das mit Wasser oder kurzkettigen Alkoholen ein azeotropes Gemisch bildet, und/oder
   (iii) thermische Behandlung des Partialglycerid-Zwischenproduktes
      beschleunigt
(b) die Enzyme vom Triglycerid über Separation oder Filtration abtrennt und
(c) die restlichen Fettsäuren und/oder deren C₁ bis C₄ -Alkylester destillativ, über Raffination oder über Extraktion vom Triglycerid entfernt, wobei man als Enzyme Lipasen, Phospholipasen oder Esterasen auswählt.

Überraschenderweise wurde gefunden, das die Umsetzung von mehrfach ungesättigten Fettsäuren und/oder deren Estern mit Glycerin zu Triglyceriden durch die Synthese unter Vakuum und zusätzlichem Zufügen eines Additivs oder Schleppmittels oder durch eine thermische Behandlung des bei der Synthese als Zwischenprodukt auftretenden Partialglyceride entscheidend beschleunigt werden kann. Die neben dem Vakuum eingesetzten Hilfsmittel führen zu einer erheblichen Reduktion der Reaktionszeiten. Verglichen mit der chemischen Synthese von Triglyceriden mehrfach ungesättigter Fettsäuren kann bei wesentlich geringeren Temperaturen gearbeitet werden, was zu einer Verminderung unerwünschter Nebenprodukte wie z.B. unerwünschter Isomere führt. Normalerweise ist die Reaktionsgeschwindigkeit dieses enzymatischen Prozesses sehr niedrig. Das erfindungsgemäße Verfahren führt jedoch zu einer Verringerung der Reaktionszeit und macht damit das enzymatische Verfahren zu einem rentablen Prozess.

Das Verfahren ist anwendbar für lineare ungesättigte Fettsäuren mit mehr als einer Doppelbindung und/oder deren C₁ bis C₄-Alkylester vorzugsweise Methyl- und/oder Ethylester auswählt aus der Gruppe, die gebildet wird von natürlich vorkommenden mehrfach ungesättigten und mehrfach konjugiert ungesättigten Fettsäuren sowie konjugierten Linol- und Linolensäuren. Vorzugsweise werden Docosahexaensäure, Eicosapentaensäure, Arachidonsäure, gamma-Linolensäure und konjugierte Linolsäure, dabei besonders bevorzugt die c9,t11 und t10,c12 Isomere der konjugierten Linolsäure (CLA) und deren Ester eingesetzt. Der gewählte Konzentrationsbereich der eingesetzten Rohstoffe liegt bei 3 bis 6 mol Fettsäure oder Ester auf 1 mol Glycerin, bevorzugt werden 3,2 bis 4,0 mol Fettsäure oder Ester pro 1 mol Glycerin eingesetzt, um eine optimale Umsetzungsgeschwindigkeit zu erreichen.

Typische Beispiele für geeignete Enzyme sind Lipasen, Phospholipasen und/oder Esterasen von Mikroorganismen die ausgewählt sind aus der Gruppe, die gebildet wird von Alcaligenes, Aspergillus, Candida, Chromobacterium, Rhizomucor, Penicilium, Pseudomonas, Rhizopus, Thermomyces, Geotrichum, Mucor, Burkholderia sowie deren Gemischen. Bevorzugt, weil besonders aktiv, sind Lipasen und Esterasen aus den Organismen Candida, Rhizomucor und Rhizopus. Besonders bevorzugt werden Candida antarctica B und Rhizomucor miehei.
Die Lipasen, Phospholipasen oder Esterasen, die ausgewählt werden, kommen bevorzugt trägergebunden zum Einsatz. Es werden insbesondere Lipasen immobilisiert auf Trägermaterial eingesetzt, speziell 3 bis 12 Gew. % Immobilisat bezogen auf den Fettanteil.
Der für die Reaktion geeignete Temperaturbereich richtet sich nach dem Aktivitätsoptimum der Enzyme. Für die bevorzugt ausgewählten Lipasen haben sich 40 bis 90° C als besonders geeignet erwiesen, speziell bevorzugt wird der Bereich von 55 bis 80°C. Dabei sollte ein Vakuum von mindestens 200 mbar, bevorzugt 1 bis 100 mbar und besonders bevorzugt 20 bis 60 mbar angelegt werden. Die bevorzugten Versuchsparameter ergeben sich aus der zu erreichenden Beschleunigung der Reaktionsgeschwindigkeit.

Überraschender Weise wurde nun festgestellt, dass durch Zusatz von bestimmten Additiven wie Komplexierungsmittel, Salzen einer schwachen Säure und Salzen von sauren Ionenaustauschern die Umsetzung entscheidend beschleunigt werden kann. Als besonders geeignet haben sich dazu Alkali- und Erdalkalisalze von Carbonaten, Citraten, Acetaten und Phosphaten erwiesen, darunter speziell Natrium- und Calciumcarbonat. Alle Zusätze werden gelöst oder als Suspension in etwas Wasser zu Beginn der Reaktion dem Ansatz zugesetzt. Ionenaustauscher können ohne Vorsuspendieren zugegeben werden.
Die Reaktion läuft in einem Konzentrationsbereich von 0,01 bis 5 Gew. % der Zusätze - bezogen auf das Gewicht der Fettsäure- bzw. der Alkylesterkomponente- optimal, bei den Salzen liegt der bevorzugte Bereich bei 0,05 bis 2 Gew. % und der besonders bevorzugte Bereich bei 0,01 bis 1 Gew. %.

Ebenso dienen Schleppmittel in Form von Lösungsmitteln, die mit Wasser oder kurzkettigen Alkoholen ein azeotropes Gemisch bilden der Reaktionsbeschleunigung. Die Schleppmittel werden kontinuierlich zum Reaktionsansatz zudosiert und über Vakuum aus dem Ansatz in gleicher Geschwindigkeit wieder entfernt.

Kombiniert man die Anwendung eines Schleppmittels mit der Anwendung eines Additivs kann man synergistische Effekte beobachten und eine optimierte Umsetzung hinsichtlich Reaktionsgeschwindigkeit und Ausbeute erreichen. Bevorzugt werden daher diese beiden Wege bei der enzymkatalysierten Herstellung von Triglyceriden , die mehrfach ungesättigte Fettsäuren enthalten, eingesetzt.

Außerdem wurde überraschender Weise festgestellt, dass die thermische Behandlung des Partialglycerides, das als Zwischenprodukt während der Umsetzung entsteht, eine weitere reaktionsbeschleunigende Massnahme darstellt. Man erhitzt dabei das Partialglycerid nach Abtrennung der immobilisierten Enzyme über Filtration bis zur einer Temperatur von 80 bis 160°C, vorzugsweise 90 bis 120°C.

Nach erfolgter Umsetzung werden die immobilisierten Enzyme durch Separation oder Filtration abgetrennt und die nicht umgesetzten Fettsäuren oder deren Alkylester durch Raffination oder Destillation, vorzugsweise Kurzwegdestillation entfernt.

### Beispiele

### Herstellung von Triglyceriden konjugierter Linolsäure

### Vergleichendes Beispiel 1

### Lipasescreening zur Synthese von Triglyceriden

In 9 Flaschen werden verschiedene Lipasen (siehe Tabelle 1, Ansätze 1-9) immobilisiert. Dazu werden jeweils gleiche Mengen der Enzympräparation und des Adsorberharzes Amberlite XAD 16 (Rohm & Haas) in der 10fachen Menge Wasser bei 30 °C über Nacht geschüttelt. Anschliessend werden die immobilisierten Enzyme abfiltriert und auf einem Papierfilter über Nacht getrocknet.
In 12 Flaschen werden verschiedene immobilisierte Lipasen (siehe Tabelle 1, Ansätze 1 - 12, Ansätze 1 - 9 sind die auf Amberlite immobilisierten Lipasen, Ansätze 10 - 12 sind bereits vom Hersteller produzierte Lipase-Immobilisate) auf ihre Fähigkeit hin Glyceride zu synthetisieren untersucht. Dazu werden 0,75 g CLA-Fettsäure, 0,07 g Glycerin, 2,5 g t-Butanol, 0,5 g Molsieb und jeweils 0,15 g der immobilisierten Lipase eingewogen. Die verschlossenen Flaschen werden bei 45 °C auf einem Schüttler bei 200 Upm für 48 h inkubiert. Der Gehalt an gebildeten Glyceriden wird gaschromatographisch analysiert und über die Peakfläche ausgewertet.
Ein analoger Versuch mit der gleichen Zusammensetzung wird bei 60 °C durchgeführt. Die 12 verschlossenen Flaschen werden bei 60 °C auf einem Schütler bei 200 Upm für 48 h inkubiert. Der Gehalt an gebildeten Glyceriden wird gaschromatographisch analysiert und über die Peakfläche ausgewertet.
Zusätzlich werden gerührte Ansätze ohne den Einsatz von t-Butanol durchgeführt. Hierzu werden in 12 Flaschen jeweils werden 0,75 g CLA-Fettsäure, 0,07 g Glycerin, 0,5 g Molsieb und 0,15 g der immobilisierten Lipase eingewogen. Die verschlossenen Flaschen werden bei 40 °C unter Rühren mit Magnetrührstäbchen für 48 h inkubiert. Der Gehalt an gebildeten Glyceriden wird gaschromatographisch analysiert und über die Peakfläche ausgewertet.

### Ergebnis:

Angegeben ist der maximal erreichte Gehalt an Glyceriden. Analysen wurden nach 24 h und 48 h durchgeführt.
A: Inkubation geschüttelt bei 45 °C
B: Inkubation geschüttelt bei 60 °C
C: Inkubation gerührt bei 40 °C

**Tab.1: Enzymatische Herstellung von Triglyceriden der CLA mit unterschiedlichen immobilisierten Lipasen**

| **Ansatz** | **Lipase** | **Hersteller** | **Organismus** | **Glyceride %** | **Glyceride %** | **Glyceride %** |
|---|---|---|---|---|---|---|
| | | | | Versuch A | Versuch B | Versuch C |
| 1 | Chirazym L 10 | Roche | Alcaligenes sp. | 0 % | 22,2 % | 21,3 % |
| 2 | Lipase A | Amano | Aspergillus niger | 1,1 % | 0,5 % | 0,6 % |
| 3 | Novocor ADL | Novozymes | Candida antarctica A | 0 % | 26,7 % | 26,8 % |
| 4 | Lipomod 34 | Biocatalysts | Candida cylindracea | 21,9 % | 9,2 % | 9,1 % |
| 5 | Lipase AY | Amano | Candida rugosa | 0 % | 22,5 % | 21,4 % |
| 6 | Lipase L115 | Biocatalysts | Porcine Pancreas | 0 % | 0 % | 0 % |
| 7 | Lipase R | Amano | Penicilium roquefortii | 1,5 % | 0 % | 0 % |
| 8 | Lipase PS | Amano | Pseudomonas cepacia | 0 % | 2,5 % | 0 % |
| 9 | Lipase F-AP 15 | Amano | Rhizopus oryzae | 60,4 % | 61,6 % | 45,0 % |
| 10 | Novozym 435 | Novozymes | Candida antarctica B | 63,1 % | 74,1 % | 63,1 % |
| 11 | Lipozym RM IM | Novozymes | Rhizomucor miehei | 47,3 % | 47,8 % | 42,1 % |
| 12 | Lipozym TL IM | Novozymes | Thermomyces lanugenosus | 41,9 % | 13,3 % | 10,3 % |

Die meisten Lipasen sind unter den gewählten Reaktionsbedingungen in der Lage Glyceride zu bilden. Unterschiede in der Syntheseleistung der Lipasen können auch durch die unterschiedlichen Unit-Aktivitäten der Enzympräparate zustande kommen. Als für die gewünschte Reaktion bevorzugtes Enzym hat sich Novozymes 435 herausgestellt.

### Beispiel 2

### Einfluss von basischen Salzen auf die Umsetzung von CLA freier Säure mit Glycerin

In 8 Kolben werden jeweils Glycerin (2,5 g) und CLA-Fettsäure (27,5 g) in einem Molverhältnis von 1 : 3,6 eingewogen. 0,33 Gew. % verschiedener Salze suspendiert in der gleichen Menge Wasser (gemäß Tabelle unten) werden zu den Ansätzen zugegeben. Nach Zugabe von 1,25 g immobilisierter Candida antarctica B Lipase (Lipase von Novozymes, Dänemark) wird bei einer Temperatur von 60 °C unter Rühren mit einem Magnetrührfisch ein Vakuum von 20 mbar angelegt. Nach 17 h wird jeweils eine Probe der Ölphase entnommen und über Säurezahlbestimmung wird der Gehalt an umgesetzter CLA Fettsäure bestimmt. Die Startsäurezahl beträgt 181.

### Ergebnis:

**Tab. 2: Beschleunigung der Umsetzung von freier CLA zu CLA-Triglyceriden gemessen an der Säurezahl in Abhängigkeit vom zugesetzten Salz**

| **Ansatz** | **Salz** | **Säurezahl** |
|---|---|---|
| 1 | Blank | 100 |
| 2 | Natriumchlorid | 132 |
| 3 | Natriumcarbonat | 46 |
| 4 | Natriumcitrat | 70 |
| 5 | Natriumacetat | 53 |
| 6 | Natriumphosphat | 64 |
| 7 | Natriumtartrat | 113 |
| 8 | Natriumtetraborat | 101 |

Die Ergebnisse zeigen, dass Natriumcarbonat, -citrat, -acetat und -phosphat die Synthese von CLA Triglycerid aus der freien Säure deutlich beschleunigen, beste Ergebnisse erreicht man mit Natriumcarbonat.

### Beispiel 3

### Abhängigkeit der Reaktionsgeschwindigkeit von der zugesetzten Natriumcarbonat Konzentration

In 3 Kolben werden jeweils Glycerin (9 g) und CLA-Fettsäure (100 g) in einem Molverhältnis von 1 : 3,7 eingewogen. Zum Ansatz 2 werden 0,1 % Natriumcarbonat suspendiert in der gleichen Menge Wasser zugegeben und zum Ansatz 3 werden 1 % Natriumcarbonat suspendiert in der gleichen Menge Wasser zugegeben. Nach Zugabe von 7 g immobilisierter Candida antarctica B Lipase wird bei einer Temperatur von 60 °C unter Rühren mit einem Magnetrührfisch ein Vakuum von 20 mbar angelegt. Nach 24 und 48 h werden jeweils Proben der Ölphase entnommen und gaschromatographisch wird der Gehalt an gebildeten CLA Glyceriden bestimmt. Angegeben ist der prozentuale Gehalt an Triglycerid bezogen auf die Summe an gebildetem Di- und Triglycerid.

### Ergebnis:

**Tab. 3: Beschleunigung der Umsetzung von freier CLA zu CLA-triglyceriden gemessen am prozentualen Gehalt an Triglycerid - bezogen auf die Summe an gebildetem Di- und Triglycerid - in Abhängigkeit von der Konzentration an zugesetztem Natriumcarbonat**

| **Ansatz** | **Konz. Natriumcarbonat** | **24 h** | **48 h** |
|---|---|---|---|
| 1 | ohne | 41 % | 63 % |
| 2 | 0,1 Gew. % | 76 % | 94 % |
| 3 | 1,0 Gew. % | 63 % | 91 % |

Natriumcarbonat zeigt insbesondere in einem Konzentrationsbereich von 0,1 % - 1,0 Gew. % eine reaktionsbeschleunigende Wirkung.

### Beispiel 4

### Einfluss von Komplexierungsmitteln auf die Umsetzung von CLA freier Säure mit Glycerin

In 5 Kolben werden jeweils Glycerin (5 g) und CLA-Fettsäure (55 g) in einem Molverhältnis von 1 : 3,6 eingewogen. 0,42 Gew% verschiedener Komplexierungsmittel suspendiert in der gleichen Menge Wasser (gemäß Tabelle unten) werden zu den Ansätzen zugegeben. Nach Zugabe von 2,5 g immobilisierter Candida antarctica B Lipase wird bei einer Temperatur von 60 °C unter Rühren mit einem Magnetrührfisch ein Vakuum von 20 mbar angelegt. Nach 17 h wird jeweils eine Probe der Ölphase entnommen und über Säurezahlbestimmung wird der Gehalt an umgesetzter CLA Fettsäure bestimmt. Die Startsäurezahl beträgt 181.

### Ergebnis:

**Tab. 4: Beschleunigung der Umsetzung von freier CLA zu CLA-triglyceriden gemessen an der Säurezahl in Abhängigkeit vom zugesetzten Komplexierungsmittel**

| **Ansatz** | **Salz** | **Säurezahl** |
|---|---|---|
| 1 | Blank | 86 |
| 2 | EDTA freie Säure | 79 |
| 3 | EDTA Di-Natriumsalz | 76 |
| 4 | EDTA Tetra-Natriumsalz | 57 |
| 5 | Trinitriloessigsäure Tri-Natriumsalz | 48 |

Insbesondere die komplett in Salzform vorliegenden Komplexierungsmittel beschleunigen die Synthese von CLA Triglycerid aus der freien Säure.

### Beispiel 5

### Einfluss von Ionentauschern auf die Umsetzung von CLA freier Säure mit Glycerin

In 8 Kolben werden jeweils Glycerin (5 g) und CLA-Fettsäure (55 g) in einem Molverhältnis von 1 : 3,6 eingewogen. 3,3 Gew% verschiedener Ionentauscher (gemäß Tabelle unten) werden zu den Ansätzen zugegeben. Nach Zugabe von 2,5 g immobilisierter Candida antarctica B Lipase wird bei einer Temperatur von 60 °C unter Rühren mit einem Magnetrührfisch ein Vakuum von 20 mbar angelegt. Nach 25 h wird jeweils eine Probe der Ölphase entnommen und gaschromatographisch wird der Gehalt an gebildeten CLA Glyceriden bestimmt. Angegeben ist der prozentuale Gehalt an Triglycerid bezogen auf die Summe an gebildetem Di- und Triglycerid.

### Ergebnis:

**Tab. 5: Beschleunigung der Umsetzung von freier CLA zu CLA-triglyceriden gemessen an der Menge des Triglycerids in Abhängigkeit vom zugesetzten Ionenaustauscher**

| **Ansatz** | **Salz** | **Triglycerid [Gew. %]** |
|---|---|---|
| 1 | Blank | 22 |
| 2 | Lewatit TP-260, Natrium-Form | 58 |
| 3 | Amberlite IRC-748, Natrium-Form | 49 |
| 4 | Lewatit MP-62, freie Base | 41 |
| 5 | Dowex MSC-1, freie Säure | 0 |
| 6 | Lewatit TP-207, Natrium-Form | 65 |
| 7 | Dowex 66, freie Base | 41 |
| 8 | Duolite C433, freie Säure | 0 |

Die Ergebnisse zeigen, dass schwach basische Ionentauscher sowie saure oder komplexierende Harze in ihrer Salzform die Synthese von CLA Triglycerid aus der freien Säure beschleunigen. Saure Ionentauscher inhibieren dagegen die Triglyceridsynthese.

### Vergleichendes Beispiel 6

### Einfluss von Stickstoffbegasung auf die Umsetzung von CLA freier Säure mit Glycerin

In 2 Kolben werden jeweils Glycerin (10,9 g) und CLA-Fettsäure (100 g) in einem Molverhältnis von 1 : 3,0 eingewogen. Nach Zugabe von 5 g immobilisierter Candida antarctica B Lipase wird bei einer Temperatur von 60 °C unter Rühren mit einem Magnetrührfisch ein Vakuum von 20 mbar angelegt. Ein Ansatz wird kontinuierlich mit Stickstoff begast. Nach 24, 72 und 96 h werden jeweils Proben der Ölphase entnommen und gaschromatographisch wird der Gehalt an gebildeten CLA Glyceriden bestimmt. Angegeben ist der prozentuale Gehalt an Triglycerid bezogen auf die Summe an gebildetem Di- und Triglycerid. Zusätzlich wird die Säurezahl zu den angegebenen Zeiten bestimmt. Der Ausgangswert der Säurezahl beträgt 179.

### Ergebnis:

**Tab. 6: Beschleunigung der Umsetzung von freier CLA zu CLA-triglyceriden gemessen am Gehalt des Triglycerids und an der Säurezahl in Abhängigkeit vom Schleppmittel Stickstoff**

| **Ansatz** | **24 h** | **72 h** | **96 h** |
|---|---|---|---|
| | Gehalt Triglycerid | | |
| | | | |
| 1 (ohne Stickstoff) | 13 % | 53 % | 63 % |
| 2 (mit Stickstoff) | 8 % | 69 % | 81 % |

| | Säurezahl | | |
|---|---|---|---|
| | | | |
| 1 (ohne Stickstoff) | 51 | 25 | 16 |
| 2 (mit Stickstoff) | 52 | 8 | 2 |

Die Begasung mit Stickstoff erhöht die Reaktionsgeschwindigkeit der Synthese von Triglycerid ausgehend von freier Fettsäure insbesondere in der zweiten Reaktionshälfte.

### Synergistischer Effekt von Stickstoffbegasung und basischem Additiv auf die Umsetzung von CLA freier Säure

In 4 Kolben werden jeweils Glycerin (5 g) und CLA-Fettsäure (55 g) in einem Molverhältnis von 1 : 3,6 eingewogen. 0,36 Gew% Natriumacetat suspendiert in der gleichen Menge Wasser werden zu den Ansätzen 3 und 4 zugegeben. Nach Zugabe von 3,0 g immobilisierter Candida antarctica B Lipase wird bei einer Temperatur von 60 °C unter Rühren mit einem Magnetrührfisch ein Vakuum von 60 mbar angelegt. Zu den Ansätzen 2 und 4 wird kontinuierlich mit Stickstoff begast. Nach 24 h werden jeweils Proben der Ölphase entnommen und gaschromatographisch wird der Gehalt an gebildeten CLA Glyceriden bestimmt.

### Ergebnis:

**Tab. 7: Beschleunigung der Umsetzung von freier CLA zu CLA-triglyceriden gemessen am Gehalt des Triglycerids in Abhängigkeit vom Schleppmittel Stickstoff und in Anwesenheit von Natriumcarbonat**

| **Ansatz** | **Stickstoff** | **Natriumacetat** | **Fettsäure** | **Monoglycerid** | **Diglycerid** | **Triglycerid** |
|---|---|---|---|---|---|---|
| 1 | Nein | Nein | 63,4 % | 12,4 % | 24,5 % | 0,0 % |
| 2 | Ja | Nein | 58,8 % | 11,9 % | 29,5 % | 0,0 % |
| 3 | Nein | Ja | 48,7 % | 3,9 % | 39,8 % | 7,8 % |
| 4 | Ja | Ja | 46,1 % | 1,2 % | 30,4 % | 22,5 % |

Auch diese Ergebnisse belegen, dass die Begasung mit Stickstoff die Reaktionsgeschwindigkeit der Synthese von Triglycerid ausgehend von freier Fettsäure entscheidend beschleunigt. Basisches Additiv und Stickstoffbegasung zeigen einen synergistischen Effekt. Der Effekt von Stickstoffbegasung zusätzlich zum angelegten Vakuum ist stärker in Anwesenheit eines basischen Additivs.

### Beispiel 8

### Einfluss des Lösungsmittels 2-Methyl-2-Butanol als Schleppmittel auf die Umsetzung von CLA freier Säure mit Glycerin

In 2 Kolben werden jeweils Glycerin (4 g) und CLA freie Säure (50 g) in einem Molverhältnis von 1 : 4,1 eingewogen. Nach Zugabe von 3 g Novozym 435 wird bei einer Temperatur von 60 °C unter Rühren mit einem Magnetrührfisch ein Vakuum von 60 mbar angelegt. In einen Ansatz wird kontinuierlich 2-Methyl-2-Butanol mit einer Flussrate von 0,05 ml/min hineingepumpt, welches unter dem angelegten Vakuum aus dem Ansatz verdampft. Nach 18 h werden jeweils Proben der Ölphase entnommen und gaschromatographisch wird der Gehalt an gebildeten CLA Glyceriden bestimmt. Angegeben ist der prozentuale Gehalt an Triglycerid bezogen auf die Summe an gebildetem Di- und Triglycerid.

### Ergebnis:

**Tab. 8: Beschleunigung der Umsetzung von freier CLA zu CLA-triglyceriden gemessen am Gehalt des Triglycerids in Abhängigkeit vom Schleppmittel 2-Methyl-2-Butanol**

| **Ansatz** | **18 h** |
|---|---|
| Gehalt Triglycerid | |
| 1 (ohne 2-Methyl-2-Butanol) | 34 % |
| 2 (mit 2-Methyl-2-Butanol) | 56 % |

Der Einsatz von 2-Methyl-2-Butanol als Schleppmittel erhöht die Reaktionsgeschwindigkeit der Synthese von Triglycerid ausgehend von CLA freier Säure.

### Beispiel 9

### Einfluss von Stickstoffbegasung auf die Umsetzung von CLA Ethylester mit Glycerin in Anwesenheit von Natriumcarbonat

In 2 Kolben werden jeweils Glycerin (5 g) und CLA-Ethylester (60 g) in einem Molverhältnis von 1 : 3,6 eingewogen. 0,15 Gew% Natriumcarbonat suspendiert in der gleichen Menge Wasser (gemäß Tabelle unten) werden zu den Ansätzen zugegeben. Nach Zugabe von 2,5 g immobilisierter Candida antarctica B Lipase wird bei einer Temperatur von 60 °C unter Rühren mit einem Magnetrührfisch ein Vakuum von 20 mbar angelegt. Ein Ansatz wird kontinuierlich mit Stickstoff begast. Nach 16, 40 und 63 h werden jeweils Proben der Ölphase entnommen und gaschromatographisch wird der Gehalt an gebildeten CLA Glyceriden bestimmt. Angegeben ist der prozentuale Gehalt an Triglycerid bezogen auf die Summe an gebildetem Di- und Triglycerid.

### Ergebnis:

**Tab. 9: Beschleunigung der Umsetzung von CLA-Ethylester mit Glycerin zu CLA-triglyceriden in Anwesenheit von Natriumcarbonat gemessen am Gehalt des Triglycerids -bezogen auf die Summe an gebildetem Di- und Triglycerid - in Abhängigkeit von der Stickstoffbegasung**

| **Ansatz** | **16 h** | **40 h** | **63 h** |
|---|---|---|---|
| Gehalt Triglycerid [Gew. %] | | | |
| 1 (ohne Stickstoff) | 17 | 20 | 21 |
| 2 (mit Stickstoff) | 36 | 65 | 77 |

Die Begasung mit Stickstoff erhöht die Reaktionsgeschwindigkeit der Synthese von Triglycerid ausgehend von CLA Ethylester über den kompletten Reaktionsverlauf. Ohne das Schleppmittel Stickstoff verläuft die Reaktion sehr langsam.

### Beispiel 10

### Einfluss von Cyclohexan-Dosierung auf die Umsetzung von CLA Methylester mit Glycerin

In 2 Kolben werden jeweils Glycerin (11 g) und CLA-Methylester (120 g) in einem Molverhältnis von 1 : 3,6 eingewogen. Nach Zugabe von 6 g immobilisierter Candida antarctica B Lipase wird bei einer Innentemperatur von 50 °C in Ansatz 1 und bei einer Innentemperatur von 55 - 60 °C in Ansatz 2 unter Rühren mit einem Magnetrührfisch ein Vakuum von 120 mbar angelegt. In beide Ansätze wird kontinuierlich mit einer Flussrate von 0,1 ml/min dosiert. Nach 20, 48 und 72 h werden jeweils Proben der Ölphase entnommen und gaschromatographisch wird der Gehalt an gebildeten CLA Glyceriden bestimmt. Angegeben ist der prozentuale Gehalt an Triglycerid bezogen auf die Summe an gebildetem Di- und Triglycerid.

### Ergebnis:

**Tab. 10: Beschleunigung der Umsetzung von CLA-Methylester mit Glycerin zu CLA-triglyceriden gemessen am Gehalt des Triglycerids - bezogen auf die Summe an gebildetem Di- und Triglycerid - in Abhängigkeit von der Zugabe des Schleppmittels Cyclohexan**

| **Ansatz** | **16 h** | **40 h** | **63 h** |
|---|---|---|---|
| Gehalt Triglycerid [Gew. %] | | | |
| 1 (50 °C innen) | 34 | 56 | 77 |
| 2 (55-60 °C innen) | 50 | 71 | 82 |

Die Dosierung von Cyclohexan fördert die Bildung von CLA Triglycerid. Zusätzlich verbessert die Erhöhung der Innentemperatur von 50 °C auf 55 - 60 °C die Bildung von Triglyceriden.

### Beispiel 11

### Reaktionsbeschleunigung durch thermische Behandlung des Partialglycerid-Zwischenproduktes bei der CLA Triglyceridsynthese

In einen Kolben werden Glycerin (25 g) und CLA-Methylester (275 g) in einem Molverhältnis von 1 : 3,4 eingewogen. 0,33 % Natriumcarbonat suspendiert in der gleichen Menge Wasser werden zugegeben. Nach Zugabe von 12,5 g immobilisierter Candida antarctica B Lipase wird bei einer Temperatur von 60 °C unter Rühren mit einem Magnetrührfisch ein Vakuum von 20 mbar angelegt. Nach 5 h Reaktionszeit wird das immobilisierte Enzym durch Filtration abgetrennt. Der Ansatz wird gaschromatographisch auf seine Glyceridverteilung analysiert und in 4 Ansätze a 50 g aufgeteilt. Nach 5 h Reaktionszeit wurde noch kein CLA Triglycerid gebildet und der Anteil der Diglyceride bezogen auf Gesamtglyceridgehalt beträgt 92 %. Der erste Ansatz wird nicht behandelt. Der zweite Ansatz wird für 30 min auf 120 °C erhitzt. Der dritte Ansatz wird mit 2 Gew % Lewatit S 100 versetzt und für 30 min auf 80 °C erhitzt. Der vierte Ansatz wird mit 0,2 Gew % Eisen-2-chlorid versetzt und für 30 min auf 80 °C erhitzt. Nach der Temperierung wird aus den Ansätzen 3 +4 Lewatit und Eisenchlorid über Filtration entfernt. Zu den Ansätzen wird je 3 g immobilisierte Candida antarctica B Lipase gegeben und bei einer Temperatur von 60 °C unter Rühren mit einem Magnetrührfisch wird ein Vakuum von 20 mbar angelegt. Nach 16 h werden jeweils Proben der Ölphase entnommen und gaschromatographisch wird der Gehalt an gebildeten CLA Glyceriden bestimmt. Angegeben ist der prozentuale Gehalt an Triglycerid bezogen auf die Summe an gebildetem Di- und Triglycerid.

### Ergebnis:

**Tab. 11: Beschleunigung der Umsetzung von CLA-Methylester mit Glycerin zu CLA-triglyceriden gemessen am Gehalt des Triglycerids nach 16 h- bezogen auf die Summe an gebildetem Di- und Triglycerid -durch thermische Behandlung des Partialglycerid-Zwischenproduktes bei der CLA Triglyceridsynthese**

| **Ansatz** | **Behandlung** | **Gehalt TG nach 16 h** |
|---|---|---|
| 1 | Blank | 34 % |
| 2 | 30 min bei 120 °C | 51 % |
| 3 | 2 % Lewatit S 100, 30 min bei 80 °C | 48 % |
| 4 | 0,2 % Eisenchlorid, 30 min bei 80 °C | 55 % |

Eine Zwischenbehandlung des gebildeten Diglycerids führt zu einer schnelleren Synthese von CLA Triglycerid. Wahrscheinlich katalysiert die Zwischenbehandlung eine Acylmigration von hauptsächlich gebildetem 1,3-Diglycerid zu 1,2-Diglycerid, welches schneller enzymatisch verestert werden kann.

### Vergleichendes Beispiel 12

### Enzymstabilisierende Eigenschaften von Silicagelpulver in wasserarmen lipasekatalysierten Reaktion bei Anwesenheit von Glycerin

Die stabilisierende Wirkung auf Candida antarctica B Lipase wird anhand der Glycerinolyse von Sonnenblumenöl zum entsprechenden Monoglycerid über einen Zeitraum von 48 Tagen gezeigt. Dazu werden 2 Flaschen mit 10 g Sonnenblumenöl, 6 g Glycerin und 7,5 g t-Butanol gefüllt. Zum Ansatz 2 werden zusätzlich noch 2 g Silikagelpulver gegeben. Nach Zugabe von 1,5 g immobilisierter Candida B Lipase werden die Ansätze auf einem Schüttler bei 45 °C inkubiert. Nach 24 h wird eine Probe entnommen und diese auf ihre Glyceridverteilung untersucht. Über einen Zeitraum von 48 Tagen werden mit den beiden Ansätzen 8 Reuses gemacht, wobei das Immobilisat vom Rest des Ansatzes über Filtration abgetrennt wird und wieder zu dem nächsten Ansatz hinzugegeben wird. Nach 48 Tagen wird von achten Ansatz ebenfalls eine Probe nach 24 h entnommen und diese auf ihre Glyceridverteilung untersucht. Angegeben ist das Verhältnis von Mono- zu Di- und Triglycerid.

**Tab. 12: Beschleunigung der Umsetzung von Sonnenblumenöl mit Glycerin gemessen an der Glyceridverteilung - in Abhängigkeit von der Zugabe des Glycerin bindenden Adsorbers Silicagelpulver**

| **Ansatz** | **Reaktionszeit** | **Monoglycerid** | **Diglycerid** | **Triglycerid** |
|---|---|---|---|---|
| 1 | 1 Tag | 80 % | 20 % | 0 % |
| 1 | 48 Tage | 60 % | 15 % | 25 % |
| 2 | 1 Tag | 80 % | 20 % | 0 % |
| 2 | 48 Tage | 83 % | 17 % | 0% |

Die Zugabe von Kieselgelpulver stabilisiert Candida antarctica B Lipase in wasserarmen Medium in Anwesenheit von Glycerinüberschüssen.
Weitere Versuche zeigten, dass insbesondere die Glycerinkonzentration einen Einfluss auf die Deaktivierung der Lipase hat. Silicagel hat die Fähigkeit Glycerin zu adsorbieren, somit verringert es die Konzentration des Glycerins in der flüssigen Phase.

### Vergleichendes Beispiel 13

### Umsetzung von CLA freier Säure mit Glycerin und Aufarbeitung des Produktes über Raffination

In 2 Kolben werden Glycerin (21,8 g) und CLA freie Säure (210 g) jeweils in einem Molverhältnis von 1 : 3,16 eingewogen. Nach Zugabe von 15 g Lipozym RM IM zu Ansatz 1 und 15 g immobilisierter Candida antarctica B Lipase zu Ansatz 2 wird bei einer Temperatur von 60 °C unter Rühren mit einem Magnetrührfisch ein Vakuum von 20 mbar angelegt. Die Ansätze werden kontinuierlich mit Stickstoff durchströmt. Nach 96 h wird die Synthese von Ansatz 1 und nach 92 h die Synthese von Ansatz 2 beendet und die immobilisierten Enzyme werden durch Filtration abgetrennt. Beide Ansätze werden mit Natriumsilikat raffiniert. Dazu werden 2 Gew.% Natriumsilikat zu den Ansätzen gegeben und diese für 1 h bei 60 °C gerührt. Anschliessend wird die Suspension über einen Papierfilter filtriert. Vor und nach der Raffination werden jeweils Proben der Ölphase entnommen und gaschromatographisch wird der Gehalt an gebildeten CLA Glyceriden bestimmt. Angegeben ist der prozentuale Gehalt an Triglycerid bezogen auf die Summe an gebildetem Di- und Triglycerid. Zusätzlich werden vor und nach der Raffination Proben entnommen und die Säurezahl wird analysiert.

**Tab. 13: Beschleunigung der Umsetzung von freier CLA zu CLA-triglyceriden gemessen am prozentualen Gehalt an Triglycerid - bezogen auf die Summe an gebildetem Di- und Triglycerid - und an der Säurezahl vor und nach Raffination.**

| **Ansatz** | **Säurezahl** | **Triglycerid** |
|---|---|---|
| | vor / nach Raffination | vor / nach Raffination |
| 1 | 12,3 / 1,5 | 96 % / 96 % |
| 2 | 2,8 / 2,1 | 93 % / 93 % |

Der Gehalt an freier CLA Fettsäure kann im CLA Triglycerid über Raffination gesenkt werden ohne das es zu einem Abbau von CLA Triglycerid kommt.

### Vergleichendes Beispiel 14

### Umsetzung von CLA Methylester mit Glycerin und Aufarbeitung des Produktes über Destillation

### Eingesetzte Rohstoffe:

| | |
|---|---|
| Glycerol: 99,9 % | 2,9 kg |
| CLA methylester: | 34,5 kg |
| Enzyme: Novozym 435, | 1,3 kg |

Apparatur: 601 Rührbehälter mit Heizvorrichung, Temperaturregelung, Vakuumanschluß inkl Regelung und Pumpe
Die eingesetzen Stoffe werden unter Rühren in den Rührbehälter gegeben und die Reaktion wird gestartet. Reaktionsbedingungen: 60 °C Innentemperatur, 50 mbar Vakuum
Nach 48 h Reaktionsdauer sind 47,9 % Triglyceride im Reaktionsgemsich gebildet worden. Diese werden mittels Kurzwegdestillation aufgereingigt.

### Bedingungen:

### Kurzwegdestillation, one step:

| | |
|---|---|
| Temperaturzuführung | 80 °C |
| Temperatur Evaporator | 190 °C |
| Temperatur Kühlungsfinger | 40 °C |
| Fluß | 200 ml/h |
| Vakuum | <0,5 mbar |

Nach Beendigung der Destillation werden 73,9 % Triglyceride erhalten.

### Vergleichendes Beispiel 15

### Umsetzung von CLA Ethylester mit Glycerin und Aufarbeitung des Produktes über Destillation

### Eingesetzte Rohstoffe:

| | |
|---|---|
| Glycerol 99,9 Gew. % | 2,9 kg |
| CLA methyl ester: | 35,5 kg |
| Enzym: Novozym 435, (Novozymes): | 1,3 kg |

Apparatur: 60 1 Rührbehälter mit Heizvorrichung, Temperaturregelung, Vakuumanschluß inkl Regelung und Pumpe
Die eingesetzen Stoffe werden unter Rühren in den Rührbehälter gegeben und die Reaktion wird gestartet. Reaktionsbedingungen: 60 °C Innentemperatur, 5 mbar Vakuum
Nach 72 h Reaktionsdauer sind 62,1 % Triglyceride im Reaktionsgemsich gebildet worden. Diese werden mittels Kurzwegdestillation aufgereingigt.

### Bedingungen:

### Kurzwegdestillation der Fettsäuren, einstufig:

| | |
|---|---|
| Temperatur Zufluss | 80 °C |
| Temperatur Evaporator | 190 °C |
| Temperatur Kühlungsfinger | 40 °C |
| Fluss | 200 ml/h |
| Vakuum | <0,5 mbar |

Nach Beendigung der Destillation werden 73,9 % Triglyceride erhalten.

## Patentansprüche

1. Verfahren zur enzymkatalysierten Herstellung von Triglyceriden, die mehrfach ungesättigte Fettsäuren enthalten, bei dem man
(a) die Umsetzung von mehrfach ungesättigten Fettsäuren und/oder deren C₁ bis C₄-Alkylestern mit Glycerin unter Vakuum in ihre Triglyceride in Gegenwart eines Enzyms durch
(i) Zugabe eines Additivs aus der Gruppe der schwach basischen Salze, Komplexierungsmittel und Salzen von sauren Ionenaustauschern und/oder
(ii) Zugabe eines Schleppmittels in Form eines Lösungsmittels, das mit Wasser oder kurzkettigen Alkoholen ein azeotropes Gemisch bildet, und/oder
(iii) thermische Behandlung des Partialglycerid-Zwischenproduktes beschleunigt
(b) die Enzyme vom Triglycerid über Separation oder Filtration abtrennt und
(c) die restlichen Fettsäuren und/oder deren C₁ bis C₄-Alkylester destillativ, über Raffination oder über Extraktion vom Triglycerid entfernt, wobei man als Enzyme Lipasen, Phospholipasen oder Esterasen auswählt.

2. Verfahren nach Anspruch 1, **dadurch gekennzeichnet, dass** man die mehrfach ungesättigte Fettsäuren und/oder deren Alkylester auswählt aus der Gruppe, die gebildet wird von Docosahexaensäure, Eicosapentaensäure, Arachidonsäure, gamma-Linolensäure, Linolsäure und konjugierter Linolsäure.

3. Verfahren nach Anspruch 2, **dadurch gekennzeichnet, dass** man als Enzyme Lipasen, Phospholipasen oder Esterasen auswählt, die trägergebunden zum Einsatz kommen.

4. Verfahren nach mindestens einem der Ansprüche 1 bis 3, **dadurch gekennzeichnet, dass** man die Umsetzung bei einem Vakuum von mindestens 200 mbar durchführt.

5. Verfahren nach mindestens einem der Ansprüche 1 bis 4, **dadurch gekennzeichnet, dass** man die Umsetzung beschleunigt durch Zugabe eines Additivs ausgewählt aus der Gruppe, die gebildet wird von Salzen eines Komplexierungsmittels, Salzen einer schwachen Säure, bevorzugt Alkali- und Erdalkalisalze von Carbonaten, Citraten, Acetaten und Phosphaten.

6. Verfahren nach mindestens einem der Ansprüche 1 bis 5, **dadurch gekennzeichnet, dass** man die Salze in Mengen von 0,001 bis 5 Gew. % bezogen auf das Gewicht der Fettsäure oder des Alkylesters einsetzt.

7. Verfahren nach mindestens einem der Ansprüche 1 bis 6, **dadurch gekennzeichnet, dass** man die thermische Behandlung des Partialglycerid-Zwischenproduktes bei 80 bis 160°C durchführt.

## Claims

1. A process for the enzyme-catalyzed production of triglycerides containing polyunsaturated fatty acids, in which
(a) the reaction of polyunsaturated fatty acids and/or C₁₋₄ alkyl esters thereof with glycerol in vacuo in the presence of an enzyme to form their triglycerides is accelerated by
(i) addition of an additive from the group of weakly basic salts, complexing agents and salts of acidic ion exchangers and/or
(ii) addition of an entraining agent in the form of a solvent which forms an azeotropic mixture with water or short-chain alcohols, and/or
(iii) heat treatment of the partial glyceride intermediate product,
(b) the enzymes are removed from the triglyceride by separation or filtration and
(c) the remaining fatty acids and/or C₁₋₄ alkyl esters thereof are removed from the triglyceride by distillation, refining or extraction, lipases, phospholipases or esterases being selected as enzymes.

2. A process as claimed in claim 1, **characterized in that** the polyunsaturated fatty acids and/or alkyl esters thereof are selected from the group consisting of docosahexaenoic acid, eicosapentaenoic acid, arachidonic acid, γ-linolenic acid, linoleic acid and conjugated linoleic acid.

3. A process as claimed in claim 2, **characterized in that** lipases, phospholipases or esterases which are used immobilized on carriers are selected as enzymes.

4. A process as claimed in at least one of claims 1 to 3, **characterized in that** the reaction is carried out in a vacuum of at least 200 mbar.

5. A process as claimed in at least one of claims 1 to 4, **characterized in that** the reaction is accelerated by addition of an additive selected from the group consisting of salts of a complexing agent, salts of a weak acid, preferably alkali and alkaline earth metal salts of carbonates, citrates, acetates and phosphates.

6. A process as claimed in at least one of claims 1 to 5, **characterized in that** the salts are used in quantities of 0.001 to 5% by weight, based on the weight of the fatty acid or the alkyl ester.

7. A process as claimed in at least one of claims 1 to 6, **characterized in that** the heat treatment of the partial glyceride intermediate product is carried out at 80°C to 160°C.

## Revendications

1. Procédé de préparation par catalyse enzymatique de triglycérides, qui comprennent des acides gras polyinsaturés, dans lequel
(a) la réaction d'acides gras polyinsaturés et/ou de leurs esters alkyliques en C₁ à C₄ avec du glycérol sous vide donnant lieu à leurs triglycérides en présence d'une enzyme est accélérée par
(i) l'addition d'un additif parmi le groupe constitué de sels faiblement basiques, d'agents complexants et de sels d'échangeurs d'ions acides et/ou
(ii) l'addition d'un agent d'entraînement sous la forme d'un solvant qui forme un mélange azéotropique avec de l'eau ou des alcools à chaîne courte, et/ou
(iii)le traitement thermique du produit intermédiaire glycéride partiel,
(b) les enzymes sont séparées du triglycéride par séparation ou filtration et
(c) le reste des acides gras et/ou de leurs esters alkyliques en C₁ à C₄ est éliminé par du triglycéride par distillation, par raffinage ou par extraction, des lipases, des phospholipases ou des estérases étant choisies en tant qu'enzymes.

2. Procédé selon la revendication 1, **caractérisé en ce que** les acides gras polyinsaturés et/ou leurs esters alkyliques sont choisis parmi le groupe qui est constitué de l'acide docosahexaénoïque, de l'acide eicosapentaénoïque, de l'acide arachidonique, de l'acide gamma-linolénique, de l'acide linoléique et de l'acide linoléique conjugué.

3. Procédé selon la revendication 2, **caractérisé en ce que** des lipases, des phospholipases ou des estérases qui sont utilisées sous forme liée à un support sont choisies en tant qu'enzymes.

4. Procédé selon au moins l'une des revendications 1 à 3, **caractérisé en ce que** la réaction est effectuée sous un vide d'au moins 200 mbar.

5. Procédé selon au moins l'une des revendications 1 à 4, **caractérisé en ce que** la réaction est accélérée par l'addition d'un additif choisi parmi le groupe qui est constitué de sels d'un agent complexant, de sels d'un acide faible, de préférence de sels de métaux alcalins et de métaux alcalino-terreux de carbonates, de citrates, d'acétates et de phosphates.

6. Procédé selon au moins l'une des revendications 1 à 5, **caractérisé en ce que** les sels sont utilisés en des quantités de 0,001 à 5 % en poids, par rapport au poids de l'acide gras ou de l'ester alkylique.

7. Procédé selon au moins l'une des revendications 1 à 6, **caractérisé en ce que** le traitement thermique du produit intermédiaire glycéride partiel est effectué entre 80 et 160°C.
